# EUROPEAN PATENT APPLICATION

(11) **EP 1 254 894 A1**
(43) Date of publication of application: **06.11.2002**
(21) Application number: 01110711.7
(22) Date of filing: 03.05.2001
(51) Int. Cl.: C07C 269/04, C07C 271/20

(54) **Process for the preparation of propamocarb**

(71) Applicant: Aventis CropScience GmbH, 65929 Frankfurt am Main (DE)
(72) Inventor: Keuchel, Johannes, Dr, 64367 Mühltal-Trautheim (DE); Schlegel, Günter, Dr, 65835 Liederbach (DE)
(74) Representative: Mérigeault, Shona

(57) **Abstract**

Propamocarb (propyl 3-(dimethylamino)propylcarbamate) by reacting propyl chloroformate in propanol

## Description

### Field of the invention

This invention relates to the preparation of propamocarb.

Propamocarb is a known fungicide having the chemical name propyl 3-(dimethylamino)propylcarbamate. The compound is normally sold as the hydrochloride salt.

The compound is normally prepared by reacting 3-(dimethylamino)propylamine with propyl chloroformate in inert solvents like toluene, ethers, etc.. We have now found that this process can be improved by carrying out the reaction at high temperatures in propanaol which can take part in the reaction.

Thus the invention provides a process for the preparation of propamocarb which comprises reacting propyl chloroformate in propanol.

using high temperatures with very high yields.

The fact that this reaction proceeds in such high yields is surprising since the propanol would be expected to react with the propyl chloroformate. especially in the presence of an acid acceptor like the 3-(dimethylamino)propylamine or propamocarb itself, (see for example Houben-Weyl, 4^{th} ed., (1983), E4, page 68). However, in terms of the propyl chloroformate a yield of up to 95% is obtained in this present process and yields based on the amine are even higher (up to 97%). As one would infer, the present invention provides high yields of propamocarb compared with processes carried out in inert solvents or water (see DE 16 43 040, DE1 567 169, GB 1 212 708, ZA 68 5172) or other standard carbamate processes (e.g. Houben-Weyl, 4^{th} ed., (1983), 8, p. 138ff and E4, p.149ff).

A particular advantage of the process of the invention is that no additional acid acceptor is required.

Another advantage is that the reaction can be carried out at a high temperature, up to boiling propanol (ca.97°C) at a higher reaction speed than in other processes using inert solvents or water (e.g. Houben-Weyl, 4^{th} ed., (1983), 8, p. 138ff and E4, p.149ff). This is surprising because often selectivity tends to decrease when using higher temperatures and in the toluene example (see experimental part) the degradation of propyl chloroformate increases significantly at temperatures higher than 65°C giving large amounts of carbon dioxide and propyl chloride.

Other advantages of the process of the present invention are that redistilled propanol can be used. Propanol is an environmentally and technically unproblematic solvent compared to ethers or dichloromethane or other inert solvents. The reaction can be carried out at a high concentration; there is almost no gas evolution during reaction and there is no waste water.

The invention is illustrated in the following example.

### Example

3-(Dimethylamino)propylamine (184.4 g) was added dropwise to recycled propanol (443.5 g) at 20-40°C. Propyl chloroformate (227.3 g) was added dropwise over 30-40 minutes. The temperature rose quickly and the flask was cooled so that the temperature was maintained at 80-85°C. After distilling off the propanol (which is later reused) the residue is propamocarb hydrochloride in a yield of 97% based on the 3-dimethylamino)-propylamine. This is 4% higher than when the reaction is carried out using toluene. The yield was 95% based on the propyl chloroformate which is at least 16% higher than when the reaction is carried out with toluene.

### Example for the toluene process:

Propyl chloroformate (180 g) was added dropwise to toluene (650 g) at 20-40°C.). 3-(dimethylamino)propylamine (125 g) was added dropwise over 30-40 minutes. The temperature rose quickly and the flask was cooled so that the temperature was maintained at 55-60°C. The reaction mixture was cooled to 40-45°C and water was added. After phase separation the crude propamocarb solution was distilled to give propamocarb-hydrochloride in a yield of 93% based on 3-(dimethylamino)-propylamine. The yield was 79% based on the propyl chloroformate.

## Claims

1. A process for the preparation of propamocarb (propyl 3-(dimethylamino)propylcarbamate) which comprises reacting propyl chloroformate in propanol.

2. A process according to claim 1 in which propanol is recycled.
